# FASCICULE DE BREVET EUROPEEN

(11) **EP 0 547 939 B1**
(45) Date de publication et mention de la délivrance du brevet: **19.03.1997**
(21) Numéro de dépôt: 92403288.1
(22) Date de dépôt: 04.12.1992
(51) Int. Cl.: B01J 8/02, B01J 8/04, B01D 3/00, C07C 41/42, C07C 41/06, C07C 2/66

(54) **Procédé de distillation réactive et appareillage pour sa mise en oeuvre**
Verfahren zur reaktiven Destillation und Vorrichtung zur Durchführung derselben
Reactive distillation process and apparatus for carrying out said process

(30) Priorité: 16.12.1991 FR 9115690
(43) Date de publication de la demande: 23.06.1993
(73) Titulaire: INSTITUT FRANCAIS DU PETROLE, 92502 Rueil-Malmaison (FR)
(72) Inventeur: Asselineau, Lionel, F-75017 Paris (FR); Mikitenko, Paul, F-78590 Noisy le Roi (FR); Viltard, Jean-Charles, F-26000 Valence (FR); Zuliani, Massimo, F-92500 Rueil Malmaison (FR)

(56) Documents cités:
- CA-A- 2 043 819
- US-A- 2 762 683
- US-A- 4 471 154
- US-A- 4 950 834

## Description

L'invention concerne un procédé et un appareillage pour effectuer à la fois des réactions chimiques et la séparation par distillation du mélange réactionnel obtenu.

Ces techniques sont connues sous la dénomination de distillation réactive ou encore de distillation catalytique lorsque la réaction chimique a lieu en présence de catalyseur.

Elles s'appliquent particulièrement à la préparation d'éthers alkyliques tertiaires tels que le méthyltertiobutyléther (MTBE), le méthyltertioamyléther (TAME), l'éthyltertiobutyléther (ETBE), ou encore l'éthyltertioamyléther (TAEE), par réaction catalytique sur l'oléfine appropriée (isobutène ou isopentène) de l'alcool approprié (méthanol ou éthanol, suivant le cas).

Elles peuvent encore s'appliquer aux réactions d'alkylation du benzène, par réaction catalytique avec l'oléfine appropriée, par exemple l'éthylène ou le propylène, pour former l'alkylbenzène correspondant, par exemple l'éthylbenzène ou l'isopropyl benzène.

En ce qui concerne plus particulièrement les réactions d'éthérification par distillation réactive, divers procédés ont déjà été proposés dans l'art antérieur, notamment dans les documents US-A-3629478, EP-A-0008860, FR-A-2503700 et FR-B-2628737.

Le brevet US-A-3629478 enseigne d'utiliser des plateaux de distillation et de ne disposer le catalyseur, en vrac (c'est-à-dire librement), que dans les descentes de liquide desdits plateaux de distillation, ceci afin d'éviter l'effet perturbateur de la phase vapeur à travers le catalyseur. Mais, la présence du catalyseur dans lesdites descentes crée une perte de charge telle que le liquide préfère descendre à contre-courant dans les orifices prévus pour la vapeur sur la table de travail de chaque plateau de distillation, plutôt que de passer à travers le catalyseur qui se trouve ainsi mis hors-circuit.

Le brevet EP-B-0008860 propose d'utiliser une colonne de distillation remplie d'un catalyseur approprié pour la préparation du méthyltertiobutyléther (MTBE), dans laquelle le catalyseur agit également comme garnissage pour la distillation, formant ainsi le MTBE et le séparant en même temps des constituants à quatre atomes de carbone. Bien que le procédé décrit dans ce brevet représente un apport technique important dans le domaine de la distillation réactive, le contact entre la phase liquide et le catalyseur semble plus ou moins intermittent du fait de l'effet perturbateur de la phase vapeur.

Le brevet FR-B-2503700 propose d'utiliser un série d'étapes catalytiques avec une circulation ascendante vapeur-liquide dans chaque lit catalytique, le catalyseur étant noyé. Mais l'effet de distillation n'est pas aussi important que prévu. De plus, un problème d'hydrodynamique peut se poser : en effet, du fait de la gravité, il ne sera pas toujours aisé pour le liquide de monter à travers chaque lit catalytique.

Le brevet FR-B-2628737 propose une technologie faisant alterner les zones de distillation et de réaction de telle manière que, dans une zone de réaction, seule la phase liquide traverse le lit catalytique, dans le sens descendant. Cette technologie présente l'inconvénient de nécessiter, pour un fonctionnement optimal, que chaque zone de distillation comprenne, outre un ou plusieurs plateaux de distillation conventionnels, un plateau de répartition et un plateau de redistribution de liquide, ce qui accroit la hauteur de la colonne et par conséquent son coût.

La demande de brevet canadien CA-A-2043819 décrit une colonne de distillation réactive comprenant une zone dans laquelle sont effectuées concurremment une réaction catalytique et la séparation par distillation des produits de ladite réaction, cette zone comportant des "plateaux" de réaction supportant un catalyseur particulaire et des plateaux de distillation, plus particulièrement du type plateaux à calottes "couplés" avec les plateaux de réaction au moyen d'une masse liquide continue immergeant, en sa zone inférieure, ledit catalyseur et, en sa zone supérieure, ledit plateau de distillation. Le plateau de réaction comporte une cheminée destinée à amener la vapeur montant de la zone située au-dessous de l'ensemble "couplé" vers un distributeur de vapeur situé au-dessous du plateau de distillation. La hauteur de la masse liquide entre le plateau de réaction et le plateau de distillation doit être suffisante pour éviter l'entraînement des particules du catalyseur. La phase liquide s'écoule de la partie supérieure de l'ensemble couplé par débordement par dessus un déversoir par l'intermédiaire d'une descente soit vers un plateau de distillation classique, soit vers un autre ensemble "couplé" situé au-dessous de l'ensemble "couplé" considéré. De même, le plateau de réaction d'un ensemble "couplé" est alimenté en phase liquide par une descente provenant, par débordement par-dessus un déversoir, soit d'un plateau de distillation classique soit d'un autre ensemble "couplé", situé au-dessous de l'ensemble "couplé" considéré.

En résumé, l'ensemble "couplé" décrit dans ce document consiste en fait en un plateau à calottes dans le fond duquel est déposé le catalyseur, la cheminée de calotte ayant été étendue pour tenir compte de la présence de la masse catalytique et de sa hauteur.

On a maintenant découvert un procédé mettant en jeu une configuration telle que les zones de réaction, alternant avec des zones de distillation sont nettement séparés de ces dernières, c'est-à-dire sans masse liquide continue entre une zone de réaction et la zone de distillation voisine. Une telle configuration, permet de s'affranchir de la présence d'une masse importante de liquide constituant une importante rétention d'eau, nécessitant une colonne plus haute et un poids plus élevé, ce qui entraîne un coût d'installation plus élevé. De plus, comme cela apparaitra dans la description qui suit, la configuration de l'invention permet une plus grande souplesse dans l'arrangement des diverses zones (réaction et distillation) dans la colonne.

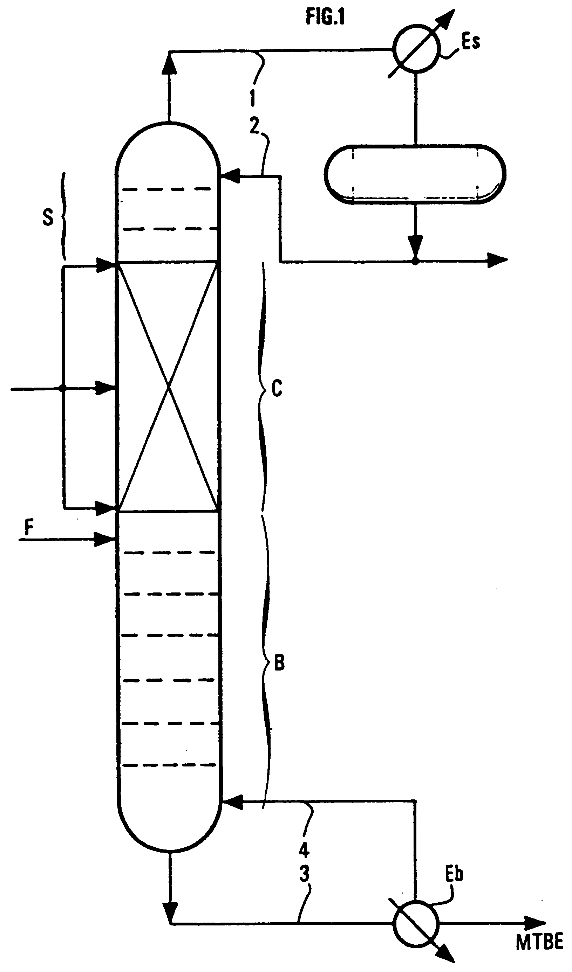

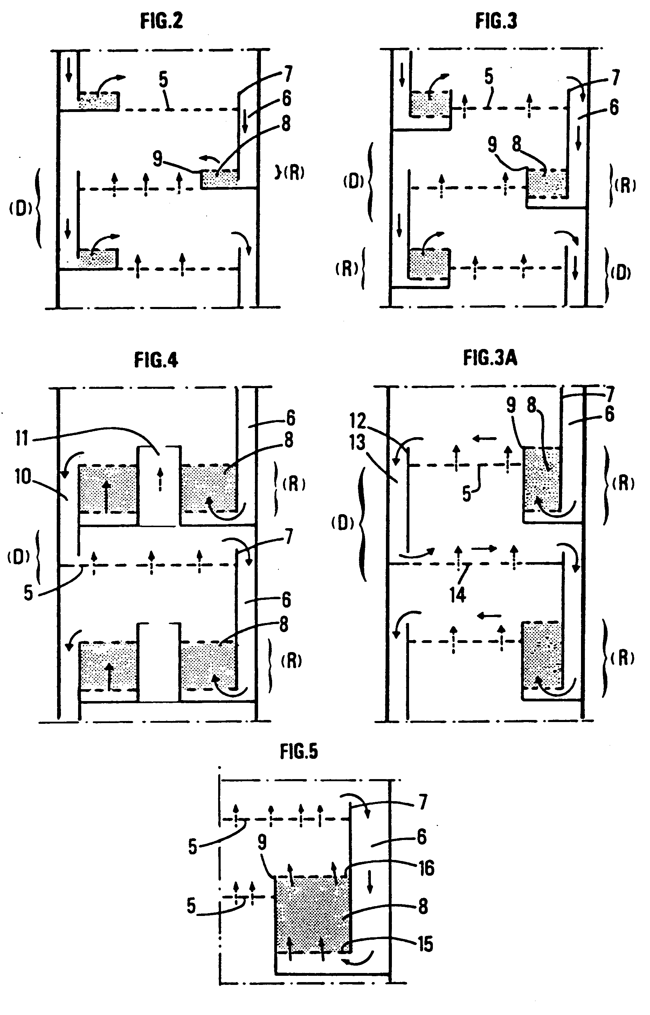

Le procédé et l'appareillage de l'invention peuvent s'appliquer à diverses réactions équilibrées, en phase liquide, pour lesquelles on peut isoler le produit de réaction par distillation dans les conditions de température et de pression auxquelles on réalise la réaction. On peut mentionner par exemple des réactions d'isomérisation de paraffines en isoparaffines, des réactions d'alkylation d'hydrocarbures aromatiques et des réactions d'éthérification entre une isooléfine (par exemple l'isobutène et l'isopentène) et un monoalcool aliphatique (tel que le méthanol ou l'éthanol).
Le procédé et l'appareillage de l'invention seront décrits ci-après en liaison avec les figures annexées, parmi lesquelles : la Figure 1 illustre sous une forme schématique l'agencement général de la colonne de distillation réactive et les Figures 2 , 3 , 3A , et 4 illustrent la configuration de cellules de distillation et de cellules réactionnelles qui composent au moins en partie la colonne de distillation réactive, selon des modes de réalisation particuliers donnés à titre d'exemples. La Figure 5 est une vue agrandie d'une cellule de distillation catalytique de la Figure 3.

D'une manière générale, le procédé de distillation réactive de l'invention est caractérisé :
- en ce qu'on introduit les réactifs séparément ou en mélange, purs ou dilués, en au moins un niveau d'une colonne de distillation réactive qui renferme :
   a) au moins une cellule réactionnelle renfermant un lit du catalyseur approprié pour la réaction considérée, et
   b) au moins une cellule de distillation constituée par au moins un plateau de distillation conventionnel ;
- chaque cellule réactionnelle étant séparée de la (ou des) cellule(s) de distillation adjacente(s) par une interface non homogène;
- en ce qu'on maintient des conditions de distillation dans ladite colonne de distillation réactive, de façon à avoir une phase liquide et une phase vapeur dans ladite colonne ;
- en ce qu'on fait circuler au moins une partie de la phase liquide du bas vers le haut à travers le catalyseur dans les cellules réactionnelles ;
- en ce qu'on fait circuler au moins une partie de la phase vapeur de la distillation du bas vers le haut, de telle manière que ladite phase vapeur soit en contact avec la phase liquide dans les cellules de distillation ;
- en ce qu'on recueille au moins une partie, de préférence la quasi totalité, du produit de réaction recherché à une seule extrêmité de la colonne de distillation réactive, généralement à la base de celle-ci ;
- et en ce qu'on recueille au moins une partie, de préférence la quasi totalité, du diluant éventuel des réactifs ainsi que du ou des réactifs éventuellement excédentaires, à l'autre extrêmité de la colonne, généralement à son sommet.

De manière préférée, dans le procédé de l'invention, on fait circuler la totalité de la phase liquide du bas vers le haut à travers le catalyseur installé dans des cellules réactionnelles.

De manière préférée également, on fait circuler la totalité de la phase vapeur de la distillation de manière qu'elle ne soit en contact avec la phase liquide que dans les cellules de distillation et non dans les cellules réactionnelles.

La colonne de distillation réactive utilisée dans le procédé de l'invention comprend essentiellement trois zones, comme représenté sur la Figure 1, à savoir : une zone de distillation catalytique proprement dite (C), dans laquelle progressent la distillation et la réaction chimique, l'une favorisant l'autre, un sommet (S) et une base (B).

Le sommet (S) de la colonne comporte au moins un plateau de distillation conventionnel ; il est équipé d'une conduite 1 pour l'évacuation des vapeurs des constituants les plus volatils et d'une conduite 2 pour l'introduction d'un reflux liquide constitué par une fraction des vapeurs condensées dans l'échangeur Es.

De même, la base (B) de la colonne comporte au moins un plateau de distillation conventionnel ; elle est équipée d'une conduite 3 pour l'évacuation des constituants les moins volatils sous forme liquide et d'une conduite 4 pour l'introduction de la vapeur de rebouillage générée par vaporisation partielle du liquide de fond, dans l'échangeur Eb. Elle est également équipée d'une ou plusieurs conduites pour l'introduction de tout ou partie des réactifs.

La zone de distillation catalytique proprement dite (C) reçoit, de manière interne, une phase liquide générée par le reflux introduit au sommet de la colonne et une phase vapeur générée par la vapeur de rebouillage introduite à la base de la colonne ainsi que, de manière externe, à au moins un niveau, un appoint éventuel d'au moins un des réactifs, purs ou dilués. Les cheminements de la phase liquide et de la phase vapeur à l'intérieur de ladite zone de distillation catalytique sont caractéristiques de l'invention. Ces cheminements sont imposés par les internes de la colonne, constitués de cellules de réaction (R) et de cellules de distillation (D) disposées de manière alternée. L'ensemble formé par une cellule de réaction (R) et la cellule de distillation (D) voisine constitue une "cellule de distillation réactive".

Les cellules de distillation réactive, caractéristiques de l'appareillage de l'invention, seront décrites ci-après en liaison avec les Figures 2 , 3 , 3A et 4, qui en illustrent diverses formes de réalisation, chacune de ces formes pouvant être choisie en fonction notamment de la quantité de catalyseur à installer, elle-même déterminée par la conversion désirée pour les réactifs.

Les cellules de distillation (D) comprennent un ou plusieurs plateaux de distillation conventionnels. Ces plateaux peuvent être choisis parmi les plateaux de distillation connus de l'homme de l'art, notamment les plateaux perforés, les plateaux à calottes, les plateaux à clapets.

En général, ces plateaux comportent chacun (Figures 2, 3, 3a ou 4) :
- au moins une table de travail discontinue, c'est-à-dire munie de discontinuités pour le passage de la phase vapeur, chaque table de travail étant destinée au brassage et mélangeage des courants liquide et vapeur,
- au moins une descente (ou goulotte) 6, par exemple située au bord de chaque plateau de distillation, pour le conditionnement du liquide et le contrôle de la régularité de son écoulement (descente à travers laquelle va s'écouler le liquide se trouvant auparavant sur la table de travail dudit plateau), et
- au moins un déversoir (ou petite margelle) 7 bordant chaque descente, destiné au maintien d'un certain niveau de liquide sur la table de travail dudit plateau et donc au contrôle de la régularité de l'évacuation du liquide de ladite table de travail.

Les cellules de réaction (R) comportent un espace de confinement 8 pour le catalyseur, adapté pour être traversé de bas en haut par la phase liquide sans être traversé par la phase vapeur de la distillation, cette dernière traversant uniquement les cellules de distillation (D), dans lesquelles elle est mise en contact avec la phase liquide, sur chaque plateau.

Le catalyseur à installer dans l'ensemble des espaces de confinement 8 peut être conditionné sous toute forme adéquate, notamment sous forme sensiblement cylindrique ou sous forme sensiblement sphérique, les dimensions des particules de catalyseur pouvant varier par exemple d'environ 0,3 à 20 mm.

Dans chaque lit catalytique, le catalyseur peut être enfermé dans une ou plusieurs enveloppes perméables au liquide mais imperméables aux particules catalytiques (c'est-à-dire ne laissant pas passer lesdites particules solides de catalyseur), enveloppes constituées par exemple par une toile en tissu, par une toile à base de fils croisés, en majeure partie métallique.

Le catalyseur peut également être disposé en vrac, c'est-à-dire librement, à l'intérieur de chaque lit catalytique. Dans ce cas, pour maintenir le catalyseur en place et pour éviter qu'il soit entraîné par le courant de liquide qui le traverse, on peut prévoir de confiner le lit catalytique entre une grille supérieure 16 et une grille inférieure 15 présentant une maille plus fine que les plus petites particules de catalyseur. Avantageusement, la grille inférieure 15 peut être légèrement surélevée par rapport au fond de la cellule de réaction (R), de manière à permettre une meilleure répartition de la phase liquide, lorsqu'elle aborde la partie inférieure du lit catalytique, comme représenté sur la Figure 5.

Lorsque le catalyseur est disposé en vrac, on peut prévoir de l'utiliser sous forme expansée ou fluidisée.

Que le catalyseur soit disposé en vrac ou enfermé dans une pluralité d'enveloppes, on pourra par exemple lui conférer un taux de vide d'au moins 1/3 pouvant aller jusqu'à 2/3.

Plus particulièrement (Figures 2 , 3 et 3A), l'espace de confinement 8 pour le catalyseur d'une cellule de réaction (R) reçoit la phase liquide contenant les réactifs s'écoulant du plateau de distillation 5 le plus bas de la cellule de distillation (D) située immédiatement en-dessus par débordement par-dessus le déversoir 7 et par la descente 6. Dans cet espace 8, à travers le lit de catalyseur, la phase liquide se déplace du bas vers le haut et se déverse sur le plateau de distillation 5 le plus haut de la cellule de distillation (D) située immédiatement en-dessous, par débordement par dessus le rebord 9 dudit espace de confinement 8.

En général, le plateau de distillation 5 de la cellule de distillation (D), lorsque celle-ci ne comprend qu'un plateau, ou le plateau de distillation 5 le plus haut de la cellule de distillation (D), lorsque celle-ci en comporte plus d'un, est contigu à la paroi de l'espace de confinement 8 de la cellule de réaction (R) qui le précède dans l'écoulement de la phase liquide. De plus, les espaces de confinement 8 pour le catalyseur peuvent être conçus de telle façon que leur fond soit au même niveau (Figure. 2) ou plus bas (Figures 3 et 3A) que le plateau de distillation 5 qui suit (dans le sens de l'écoulement du liquide).

Dans le cas où une même cellule de distillation (D) comporte plus de deux plateaux de distillation (voir Figure 3A), tout plateau supérieur 5 est relié au plateau immédiatement inférieur 14 par au moins une descente 13. La phase liquide s'écoule alors par débordement par dessus le déversoir 12. Par ailleurs, dans les modes de réalisations décrits ci-dessus en liaison avec les Figures 2, 3 et 3A, la phase vapeur de la distillation circulant de bas en haut passe d'une cellule de distillation (D) à la cellule de distillation supérieure sans rencontrer d'obstacle, la cellule de réaction (R) intermédiaire n'occupant par son espace de confinement 8 qu'une faible partie de la section de la colonne.

De manière différente, dans un mode de réalisation tel que celui représenté par la Figure 4, où les cellules de réaction (R) occupent la quasi totalité de la section de la colonne, le plateau de distillation 5 le plus élevé de la cellule de distillation (D) qui suit une cellule de réaction (R) ne peut plus être contigu à la paroi de l'espace de confinement 8 de celle-ci, mais doit être placé au-dessous de ladite cellule de réaction (R) et l'on prévoit au moins une descente 10 pour l'écoulement de la phase liquide de la cellule de réaction (R) vers ledit plateau de distillation 5, ainsi que, pour le passage de la phase vapeur de la distillation, au moins une cheminée 11 ménagée à travers l'espace de confinement 8 de ladite cellule de réaction (R). On fait appel à une telle configuration lorsque l'on désire disposer une grande quantité de catalyseur dans les cellules de réaction (R).

La caractéristique essentielle du procédé et du dispositif de l'invention est que chaque cellule réactionnelle est séparée de la (ou des) cellule(s) de distillation adjacente(s). La séparation par une interface non-homogène est constituée, par exemple selon l'arrangement des cellules réactionnelles, par la paroi latérale et/ou le fond de celles-ci, qui empêchent tout transfert direct de matière entre l'intérieur des cellules réactionnelles et les zones de distillation voisines. En particulier, cette séparation implique qu'il n'y a pas de jonction liquide continue entre les deux types de cellules.

Il doit être entendu dans l'invention que, dans une même zone de distillation catalytique (C), il est possible de combiner la mise en place de cellules de distillation réactive de types différents suivant le niveau de la zone de distillation auquel on se trouve.

Ainsi, par exemple, on peut disposer des cellules catalytiques telles que celles représentées sur la Figure 3 dans la partie supérieure de la zone de distillation catalytique et des cellules du type représenté sur la Figure 4 dans la partie inférieure de cette même zone.

Lors du fonctionnement de la colonne, en raison de l'entraînement possible par le liquide de fines du catalyseur, il peut être avantageux de prévoir de collecter sur un plateau de soutirage le liquide s'écoulant de la cellule de réaction (R) située le plus bas dans la zone de distillation catalytique (C), de soutirer ce liquide et de le filtrer dans au moins un dispositif de filtrage (situé à l'extérieur de la colonne), et de le réintroduire dans la colonne de distillation catalytique entre la position d'où il a été soutiré et le plateau de distillation placé immédiatement au-dessous du plateau de soutirage. Ce filtrage permet d'éliminer tous les fragments éventuels de catalyseur entraînés par la phase liquide et, en particulier, les empêche de venir obturer le fond de la colonne.

Le procédé et l'appareillage de l'invention présentent notamment l'avantage de ne pas nécessiter d'intercaler des plateaux de redistribution de liquide entre chaque cellule réactionnelle et la cellule de distillation inférieure, ce qui représente une simplification et une économie lors de réalisation des colonnes.

Un autre avantage réside dans le fait que le catalyseur est noyé dans le liquide et non perturbé par la phase vapeur de la distillation, ce qui lui permet de travailler dans des conditions d'efficacité optimale.

En outre, la mise en oeuvre d'un courant ascendant de phase liquide à travers les lits de catalyseur amène une expansion et une certaine mobilité de ces lits ainsi que l'entraînement et l'évacuation des fines avec la phase liquide, ce qui permet d'éviter de colmater les lits catalytiques.

Par ailleurs, dans le cas de réactions exothermiques, par exemple pour la synthèse des éthers alkyliques tertiaires, un avantage du procédé de l'invention est que la circulation en courant ascendant de la phase liquide à travers les lits de catalyseur permet en outre d'évacuer aisément la phase vapeur générée sur le catalyseur par la chaleur de réaction.

Le procédé de l'invention et l'appareillage utilisé pour sa mise en oeuvre, tels qu'ils ont été décrits précédemment, s'appliquent plus particulièrement aux réactions d'éthérification entre une isooléfine (par exemple l'isobutène ou l'isopentène) et un monoalcool aliphatique (par exemple le méthanol ou l'éthanol) pour former les éthers correspondants. On décrit plus spécifiquement ci-après un exemple d'application à la synthèse du méthyltertiobutyléther.

La charge contenant de l'isobutène est en général constituée d'une coupe C₄ provenant notamment d'un vapocraquage, d'un craquage catalytique ou de la déshydrogénation de l'isobutane. Dans une première zone de réaction, elle est mise en contact avec du méthanol, dans des conditions réactionnelles bien connues dans l'art. Cette réaction, équilibrée, permet ce convertir une partie de l'isobutène (en général de 70 à 90 %) en MTBE. C'est le mélange issu de cette première zone qui est ensuite traité selon le procédé de distillation réactive de l'invention.

On opère dans une colonne telle que représentée schématiquement sur la Figure 1. En général, la charge F contenant de l'isobutène et du méthanol non convertis est introduite à un niveau légèrement inférieur à la zone de distillation catalytique proprement dite (C). Un appoint de méthanol est en général introduit juste au-dessus de ladite zone (C). On peut également prévoir une introduction de cet appoint en plusieurs points répartis au long de la zone de distillation catalytique.

On utilise en général un catalyseur du type résine sulfonée, par exemple une résine polystyrène-divinylbenzène sulfonée, dont la granulométrie est par exemple de 0,3 à 1,2 mm. Ce catalyseur est placé dans les cellules réactionnelles de la colonne comme décrit plus haut.

L'opération de distillation réactive est menée dans des conditions convenables pour que, au sommet de la colonne, sortent par la ligne 1 les hydrocarbures de la charge qui n'ont pas réagi, et en fond de la colonne, par la ligne 3, le méthyltertiobutyléther recherché.

Les conditions habituellement mises en oeuvre sont une pression allant par exemple d'environ 4 à 16 bars. En fonction de la pression choisie, la température de fond de la colonne peut être d'environ 110 à 170 °C et la température de tête d'environ 40 à 90 °C. On maintient en général un taux de reflux par rapport au distillat compris entre 0,5:1 et 5:1. Par ce procédé il est possible de convertir la quasi totalité de l'isobutène et d'obtenir du MTBE avec une pureté élevée, en général d'au moins 98% en moles.

L'exemple suivant illustre l'invention.

### EXEMPLE :

Dans cet exemple, on traite par le procédé de l'invention une charge issue d'un réacteur d'éthérification ayant la composition pondérale donnée dans la deuxième colonne du Tableau 1.

**TABLEAU 1**

| Constituant | Charge | Distillat | Résidu |
|---|---|---|---|
| Isobutène | 4,6 | 1,4 | <0,02 |
| Butènes | 33,7 | 76,7 | <0,2 |
| Butanes | 8,1 | 18,3 | <0,2 |
| Méthanol | 1,4 | 3,6 | - |
| MTBE | 52 | <0,1 | >99 |
| Divers | 0,2 | - | 0,6 |

On utilise une colonne en acier inoxydable de hauteur 350 cm. et de diamètre 5 cm.. Elle présente une le zone supérieure comportant 7 plateaux de distillation perforés avec déversoir, une zone de distillation réactive comportant 12 cellules de distillation réactive réalisées selon le principe schématisé sur la figure 4 et contenant chacune 5 cm³ d'un catalyseur en vrac consistant en une résine sulfonée (résine polystyrène-divinylbenzène sulfonée vendue par ROHM & HAAS sous la marque déposée Amberlyst 15), et une zone inférieure comportant 16 plateaux de distillation perforés à déversoir.

La charge est alimentée, sous un débit de 1800 g/h. au niveau du vingt-neuvième plateau (compté de haut en bas). On injecte également un appoint de méthanol, sous un débit de 43 g/h., sur le premier plateau réactif à partir du haut.

La colonne est mise en régime sous une pression de 8 bars avec un taux de reflux de 1 :1 et régulée de manière à sortir finalement en distillat les hydrocarbures inertes dans la réaction chimique ainsi que les traces de réactifs n'ayant pas réagi. Il s'établit dans la colonne un profil de température allant de 61 °C en tête à 140 °C en fond.

On soutire en tête de colonne, avec un débit de 789 g/h, un distillat ayant la composition indiquée au Tableau 1, et en fond, avec un débit de 1050 g/h, le MTBE produit ayant la composition pondérale également indiquée dans le Tableau 1.

## Revendications

1. Procédé de distillation réactive dans lequel :
- on introduit les réactifs séparément ou en mélange, purs ou dilués, en au moins un niveau d'une colonne de distillation réactive qui renferme :
(a) au moins une cellule réactionnelle renfermant un lit du catalyseur approprié ; et
(b) au moins une cellule de distillation constituée par au moins un plateau de distillation ;
- on maintient des conditions de distillation dans ladite colonne de façon à avoir une phase liquide et une phase vapeur dans ladite colonne ;
- on fait circuler au moins une partie de la phase liquide du bas vers le haut à travers le catalyseur dans chaque cellule réactionnelle;
- on fait circuler au moins une partie de la phase vapeur de la distillation du bas vers le haut à travers chaque cellule de distillation, de telle manière que ladite phase vapeur soit en contact avec la phase liquide dans les cellules de distillation;
- on recueille au moins une partie du produit recherché à une extrêmité de ladite colonne de distillation réactive; et
- on recueille au moins une partie du diluant éventuel des réactifs et de tout excès de réatif(s) à l'autre extrêmité de ladite colonne;
ledit procédé étant caractérisé en ce que chaque cellule réactionnelle n'a aucune jonction liquide continue avec la cellule de distillation adjacente.

2. Procédé selon la revendication 1, caractérisé en ce que :
- l'on fait circuler la totalité de la phase liquide du bas vers le haut à travers le catalyseur dans chaque cellule réactionnelle :
- on fait circuler la totalité de la phase vapeur de la distillation du bas vers le haut à travers chaque cellule de distillation de telle manière que ladite phase vapeur ne soit en contact avec la phase liquide que dans les cellules de distillation et non dans les cellules réactionnelles ;
- on recueille la quasi totalité du produit recherché à une extrêmité de la colonne de distillation réactive ; et
- on recueille la quasi totalité du diluant éventuel des réactifs et de tout excès de réactif(s) à l'autre extrêmité de ladite colonne.

3. Procédé selon l'une des revendications 1 et 2, caractérisé en ce qu'on met en oeuvre une réaction d'éthérification à partir d'une isooléfine et d'un monoalcool aliphatique pour former un éther alkylique tertiaire.

4. Procédé selon la revendication 3, caractérisé en ce que ladite monooléfine est l'isobutène ou l'isopentène et ledit monoalcool aliphatique est le méthanol ou l'éthanol, et l'on forme le méthyltertiobutyléther, l'éthyltertiobutyléther, le méthyltertioamyléther ou l'éthyltertioamyléther.

5. Procédé selon la revendication 1, caractérisé en ce qu'on met en oeuvre une réaction d'alkylation du benzène par une oléfine choisie parmi l'éthylène et le propylène.

6. Procédé selon l'une des revendications 1 à 5, caractérisé en ce que, dans chaque cellule réactionnelle, le catalyseur est disposé en vrac.

7. Procédé selon l'une des revendications 1 à 5, caractérisé en ce que, dans chaque cellule réactionnelle, le catalyseur est enfermé dans au moins une enveloppe perméable au liquide mais imperméable aux particules catalytiques.

8. Procédé selon la revendication 7, caractérisé en ce que le catalyseur est expansé dans chaque cellule réactionnelle.

9. Procédé selon l'une des revendications 6 à 8, caractérisé en ce que l'on maintient un taux de vide d'1/3 à 2/3 dans chaque cellule réactionnelle.

10. Appareillage pour mettre en oeuvre un procédé selon l'une des revendications 1 à 9, consistant essentiellement en une colonne de distillation réactive qui comprend une zone de distillation catalytique (C), un sommet (S) et une base (B), dans lequel ledit sommet comporte au moins un plateau de distillation conventionnel et est équipé d'une conduite 1 pour l'évacuation sous forme vapeur des produits les plus volatils et d'une conduite 2 pour l'introduction d'un reflux liquide ; ladite base (B) comporte au moins un plateau de distillation conventionnel, est équipée d'une conduite 3 pour l'évacuation sous forme liquide des produits les moins volatils et d'une conduite 4 pour l'introduction d'une vapeur de rebouillage; dans lequel ladite zone de distillation catalytique (C) comprend une succession de cellules de distillation réactive chacune formée d'une cellule de distillation (D) et d'une cellule réactionnelle (R) séparées, ladite cellule de distillation (D) comprenant au moins un plateau de distillation 5, et ladite cellule réactionnelle (R) comportant un espace de confinement 8 pour le catalyseur, adapté à être traversé par la phase liquide de bas en haut sans être traversé par la phase vapeur de la distillation; et caractérisé en ce que chaque cellule réactionnelle n'a aucune jonction liquide continue avec la cellule de distillation adjacente.

11. Appareillage selon la revendication 10, caractérisé en ce que les cellules de distillation (D) et les cellules réactionnelle (R) sont agencées de telle manière que chaque espace de confinement 8 d'une cellule réactionnelle (R) reçoive la phase liquide s'écoulant du plateau de distillation 5 le plus bas de la cellule de distillation (D) située immédiatement au dessus de ladite cellule réactionnelle (R), par un déversoir 7 et une descente 6, et que, après traversée de bas en haut du catalyseur, la phase liquide se déverse par dessus le rebord 9 dudit espace de confinement 8, sur le plateau de distillation 5 le plus haut de la cellule de distillation (D) située immédiatement en dessous.

12. Appareillage selon la revendication 11, caractérisé en ce que les espaces de confinement 8 du catalyseur n'occupant pas toute la section de la colonne, le plateau de distillation 5 le plus haut d'une cellule de distillation (D) est contigu à la paroi de l'espace de confinement 8 de la cellule de réaction (R) immédiatement supérieure.

13. Appareillage selon la revendication 11, caractérisé en ce que, un espace de confinement 8 d'une cellule de réaction (R) occupant la quasi totalité de la section de la colonne, le plateau de distillation 5 le plus haut de la cellule de distillation (D) immédiatement inférieure est placé au-dessous de ladite cellule de réaction (R), ladite cellule de réaction (R) comporte au moins une descente 10 pour l'écoulement de la phase liquide vers ledit plateau de distillation 5 et au moins une cheminée 11 pour le passage de la vapeur de distillation à travers ladite cellule de réaction (R).

14. Appareillage selon l'une des revendications 10 à 13, caractérisé en ce que au moins une cellule de distillation (D) comporte au moins deux plateaux de distillation superposés, tout plateau supérieur 5 étant relié au plateau immédiatement inférieur 14 par au moins une descente 13.

15. Appareillage selon l'une des revendications 10 à 14, caractérisé en ce que le lit catalytique est maintenu dans chaque espace de confinement 8 par une grille d'entrée 15 et une grille de sortie 16.

## Claims

1. Reactive distillation process, in which
- the reagents are introduced separately or in mixed form, pure or in diluted form, at at least one level of a reactive distillation column containing:
(a) at least one reaction cell containing an appropriate catalyst bed and
(b) at least one distillation cell constituted by at least one distillation tray,
- distillation conditions are maintained in said column so as to have a liquid phase and a vapour phase therein,
- at least part of the liquid phase is circulated from bottom to top through the catalyst in each reaction cell,
- at least part of the vapour phase of the distillation is circulated from bottom top through each distillation cell, so that said vapour phase is in contact with the liquid phase in the distillation cells,
- at least part of the sought product is collected at one end of said reactive distillation column and
- at least part of any diluent of the reagents and any excess of reagents is collected at the other end of said column;
said process being characterized in that each reaction cell has not liquid junction with the adjacent distillation cell.

2. Process according to claim 1, characterized in that:
all the liquid phase is circulated from bottom to top through the catalyst in each reaction cell,
all the vapour phase of the distillation is circulated from bottom to top through each distillation cell in such a way that said vapour phase is only in contact with the liquid phase in the distillation cells and not in the reaction cells,
almost all the sought product is collected at one end of the reactive distillation column and
almost all any diluent of the reagents and any excess of reagents is collected at the other end of said column.

3. Process according to either of the claims 1 and 2, characterized in that an etherification reaction is carried out on the basis of an isoolefin and an aliphatic monoalcohol in order to form a tertiary alkyl ether.

4. Process according to claim 3, characterized in that said monoolefin is isobutene or isopentene and said aliphatic monoalcohol is methanol or ethanol and methyl tert. butyl ether, ethyl tert. butyl ether, methyl tert. amyl ether or ethyl tert. amyl ether is formed.

5. Process according to claim 1, characterized in that a reaction of alkylating benzene by an olefin chosen from among ethylene and propylene is performed.

6. Process according to any one of the claims 1 to 5, characterized in that in each reaction cell the catalyst is arranged loosely.

7. Process according to any one of the claims 1 to 5, characterized in that in each reaction cell, the catalyst is enclosed in at least one liquid-permeable, but catalytic particle-impermeable envelope.

8. Process according to claim 7, characterized in that the catalyst is expanded in each reaction cell.

9. Process according to any one of the claims 6 to 8, characterized in that a vacuum level of 1/3 to 2/3 is maintained in each reaction cell.

10. Apparatus for performing the process according to any one of the claims 1 to 9 essentially consisting of a reactive distillation column comprising a catalytic distillation zone (C), a top (S) and a base (B), in which said top has at least one conventional distillation tray and is provided with a pipe (1) for the discharge in vapour form of the most volatile products and a pipe (2) for the introduction of a liquid reflux; said base (B) has at least one conventional distillation tray and is equipped with a pipe (3) for the discharge in liquid form of the least volatile products and a pipe (4) for the introduction of a reboiling vapour; in which said catalytic distillation zone (C) comprises a succession of reactive distillation cells, each formed of separated distillation cell (D) and reaction cell (R), said distillation cell (D) having at least one distillation tray (5) and said reaction cell (R) having a confinement space (8) for the catalyst and which can be traversed by the liquid phase from bottom to top without being traversed by the vapour phase of the distillation; and characterized in that each reaction cell has not continuous liquid function with the adjacent distillation cell.

11. Apparatus according to claim 10, characterized in that the distillation cells (D) and the reaction cells (R) are arranged in such a way that each confinement space (8) of a reaction cell (R) receives the liquid phase flowing from the lowest distillation tray (5) of the distillation cell (D) located immediately below, said reaction cell (R) by an overflow (7) and a down pipe (6) and that, after traversing the catalyst from top to bottom, the liquid phase flows over the ledge (9) of said confinement space (8) onto the highest distillation tray (5) of the distillation cell (D) located immediately blow the same.

12. Apparatus according to claim 11, characterized in that the confinement spaces (8) of the catalyst do not occupy the entire cross-section of the column, so that the highest distillation tray (5) of a distillation cell (D) is contiguous with the wall of the confinement space (8) of the reaction cell (R) immediately above it.

13. Apparatus according to claim 11, characterized in that, as the confinement space (8) of a reaction cell (R) occupies almost the entire column cross-section, the highest distillation tray (5) of the distillation cell (D) immediately blow it is positioned blow said reaction cell (R), the latter having at least one down pipe (10) for the outflow of the liquid phase towards said distillation tray (5) and at least one stack (11) for the passage of the distillation vapour through said reaction cell (R).

14. Apparatus according to any one of the claims 10 to 13, characterized in that at least one distillation cell (D) has at least two superimposed distillation trays, any upper tray (5) being connected to the tray (14) immediately below it by at least one down pipe (13).

15. Apparatus according to any one of the claims 10 to 14, characterized in that said catalyst bed is maintained in each confinement space (8) by an entrance grating (15) and an exit grating (16).

## Patentansprüche

1. Verfahren für die Reaktions-Destillation, in dem
- die Reagenzien getrennt oder gemischt, rein oder verdünnt, an mindestens einem Punkt einer Kolonne für die Reaktions-Destillation eingeführt werden, die
a) mindestens eine Reaktionszelle mit einer Schicht des geeigneten Katalysators und
b) mindestens eine Destillationszelle aus mindestens einem Destillationsboden umfaßt;
- solche Destillationsbedingungen in der besagten Kolonne für die Reaktions-Destillation erhalten bleiben, daß in der besagten Kolonne eine Flüssigphase und eine Dampfphase vorhanden sind;
- mindestens ein Teil der Flüssigphase von unten nach oben durch den Katalysator in jeder Reaktionszelle hochwandert;
- mindestens ein Teil der Destillationsdampfphase von unten nach oben durch jede Destillationszelle emporsteigt, damit die besagte Dampfphase in Berührung mit der Flüssigphase der Destillationszellen kommt;
- das gewünschte Reaktionsprodukt mindestens zum Teil, an einem Ende der reaktiven Destillationskolonne abgezogen wird und
- das Lösungsmittel für die Reagenzien sowie die möglicherweise überschüssige(n) Reagenz(ien) am anderen Ende der besagten Kolonne abgezogen werden,
wobei das Verfahren dadurch gekennzeichnet ist, daß keine Reaktionszelle eine Dauerverbindung mit der angrenzenden Destillationszelle über Flüssigkeit erfährt.

2. Verfahren nach Anspruch 1 dadurch gekennzeichnet, daß
- die gesamte Flüssigphase von unten nach oben durch den Katalysator in jeder Reaktionszelle fließt;
- die gesamte Destillationsdampfphase von unten nach oben durch jede Destillationszelle so geleitet wird, daß die besagte Dampfphase lediglich in den Destillationszellen aber nicht in den Reaktionszellen in Berührung mit der Flüssigphase kommt;
- das gewünschte Produkt fast ganz an einem Ende der Kolonne für die Reaktions-Destillation und
- das mögliche Lösungsmittel für die Reagenzien sowie jeder Reagenz(ien)-Überfluß fast ganz am anderen Ende der besagten Kolonne entnommen werden.

3. Verfahren nach einem der Ansprüche 1 und 2 dadurch gekennzeichnet, daß eine Veretherungsreaktion zwischen einem Isoolefin und einem einwertigen aliphatischen Alkohol eingeleitet wird, um einen tertiären Alkylether zu bilden.

4. Verfahren nach dem Anspruch 3 dadurch gekennzeichnet, daß das besagte Monoolefin Isobuten oder Isopenten ist, und der einwertige aliphatische Alkohol Methanol oder Ethanol, woraus Tert-Butylmethylether, Tert-Butylethylether, Tert-Amylmethylether oder Tert-Amylethylether entstehen.

5. Verfahren nach Anspruch 1 dadurch gekennzeichnet, daß eine Alkylierungsreaktion des Benzols durch ein Olefin (Ethylen oder Propylen) eingeleitet wird.

6. Verfahren nach einem der Ansprüche 1 bis 5 dadurch gekennzeichnet, daß der Katalysator innerhalb jeder Reaktionszelle lose ist.

7. Verfahren nach einem der Ansprüche 1 bis 5 dadurch gekennzeichnet, daß der Katalysator in mindestens einer für die Flüssigkeit durchlässigen aber für die Katalysatorpartikeln undurchlässigen Umhüllung eingeschlossen ist.

8. Verfahren nach Anspruch 7 dadurch gekennzeichnet, daß der Katalysator in jeder Reaktionszelle aufgeschäumt wird.

9. Verfahren nach einem der Ansprüche 6 bis 8 dadurch gekennzeichnet, daß ein Vakuumsanteil von 1/3 bis 2/3 in jeder Reaktionszelle herrscht.

10. Einrichtung für die Anwendung des Verfahrens nach einem der Ansprüche 1 bis 9, die vorwiegend aus einer Kolonne für die Reaktions-Destillation besteht, die einen katalytischen Destillationsbereich (C), eine Spitze (S) und einen Boden (B) umfaßt, in der die besagte Spitze mindestens einen herkömmlichen Destillationsboden enthält und mit einer Leitung 1 für den Abzug als Dampf der flüchtigsten Produkte, mit einer Leitung 2 für die Einführung des Rücklaufes ausgerüstet ist; der besagte Boden (B) enthält mindestens einen herkömmlichen Destillationsboden und ist mit einer Leitung 4 für die Zuführung des Abkochdampfes versehen; in der der besagte reaktive Destillationsbereich (C) eine Reihe von relativen Destillationszellen aufweist, die je aus einer Destillationszelle (D) und einer getrennten Reaktionszelle (R) bestehen, wobei die besagte Destillationszelle (D) mindestens einen Destillationsboden 5 und die besagte Reaktionszelle einen Aufnahmebereich 8 für den Katalysator, der dazu geeignet ist, von der Flüssigphase von unten nach oben aber nicht von der emporsteigenden Destillationsdampfphase durchwandert zu werden, enthalten; dadurch gekennzeichnet, daß keine Reaktionszelle eine Dauerverbindung mit der angrenzenden Destillationszelle über Flüssigkeit erfährt.

11. Einrichtung nach Anspruch 10 dadurch gekennzeichnet, daß die Destillationszellen (D) und die Reaktionszellen (R) so angeordnet sind, daß jeder Aufnahmebereich 8 einer Reaktionszelle (R) über einen Überlauf 7 und ein Fallrohr 6 die Flüssigphase erhält, die vom tiefstgelegenen Destillationsboden 5 der Destillationszelle (D), die unmittelbar oberhalb der besagten Reaktionszelle (D) liegt, abfließt, und daß die Flüssigphase, nachdem sie den Katalysator von unten nach oben durchwandert hat, über den Rand 9 des besagten Aufnahmebereiches 8 auf den höchstgelegenen Destillationsboden 5 der unmittelbar darunterliegenden Destillationszelle (D) abfließt.

12. Einrichtung nach Anspruch 11 dadurch gekennzeichnet, daß die Katalysatoraufnahmebereiche 8 den ganzen Kolonnenquerschnitt nicht in Anspruch nehmen, wodurch der in der Destillationszelle (D) höchstgelegene Destillationsboden 5 an die Wand des Aufnahmebereiches 8 der unmittelbar darüberliegenden Reaktionszelle (R) angrenzt.

13. Einrichtung nach Anspruch 11 dadurch gekennzeichnet, daß ein Aufnahmebereich 8 einer Reaktionszelle (R) beinahe den gesamten Kolonnenquerschnitt in Anspruch nimmt, wodurch der Destillationsboden 5 in der direkt darunterliegenden Destillationszelle (D) sich unterhalb der besagten Reakionszelle (R) befindet, wodurch die besagte Reaktionszelle (R) mindestens ein Fallrohr 10 für den Abfluß der Flüssigphase zu dem besagten Destillationsboden 5 und mindestens einen Abzug 11 für den Destillationsdampfabzug durch die besagte Reaktionszelle aufweist.

14. Einrichtung nach einem der Ansprüche 10 bis 13 dadurch gekennzeichnet, daß mindestens eine Destillationszelle (D) mindestens zwei übereinanderliegende Böden enthält, wobei der obere Boden 5 mit dem unmittelbar darunterliegenden Boden 14 durch mindestens ein Fallrohr 13 verbunden ist.

15. Einrichtung nach einem der Ansprüche 10 bis 14 dadurch gekennzeichnet, daß die Katalysatorschicht in jedem Aufnahmebereich 8 durch einen Eingangsgitterrost 15 und einen Ausgangsgitterrost 16 festgehalten wird.
